# EUROPEAN PATENT APPLICATION

(11) **EP 4 238 549 A1**
(43) Date of publication of application: **06.09.2023**
(21) Application number: 23159351.8
(22) Date of filing: 01.03.2023
(51) Int. Cl.: A61K 8/64, A61K 8/9722, A61Q 19/08

(54) **ANTI-AGING COMPOSITION, SKIN CARE PRODUCT AND COSMETIC**

(30) Priority: 02.03.2022 CN 202210202557
(71) Applicant: Galenic Cosmetics Laboratory, 75017 Paris (FR)
(72) Inventor: LU, Wangwang, GUANGDONG, 510275 (CN); WANG, Mengping, GUANGDONG, 510275 (CN); GE, Xinye, GUANGDONG, 510275 (CN); LU, Yuan, GUANGDONG, 510275 (CN)
(74) Representative: Hirsch & Associés

(57) **Abstract**

The present invention provides an anti-aging composition, which comprises *Chlamydomonas* extract and acetyl hexapeptide-8 in a mass ratio of 1:2000-5:2. The present invention also provides use of the anti-aging composition, and a skin care product and cosmetic comprising the anti-aging composition. This application creatively uses *Chlamydomonas* extract and acetyl hexapeptide-8 in combination, especially Rheolique and Acti-Klotho, which can inhibit inflammation, promote autophagy, and inhibit collagen degradation, and can continuously promote collagen production, and reduce the secretion of destructive proteases and inflammatory factors, finally achieving the increase of collagen, thereby fundamentally improving the condition of aging skin.

## Description

### FIELD

The present invention relates to the field of daily cosmetics, and in particular to an anti-aging composition, skin care product and cosmetic.

### BACKGROUND

Aging is an irresistible and irreversible natural phenomenon that is always accompanied by various degenerative diseases and unsightly skin conditions. The pursuit of beauty in human nature makes anti-aging cosmetics always receive widespread attention.

In the first generation of anti-aging products, active substances with film-forming property achieve anti-wrinkle effects by forming a film on the skin and reducing the pulling of muscles. However, they have obvious shortcomings, for example, they have short-term action time and do not fundamentally improve the skin.

With the further development of modern technology, scientists have discovered that the condition of aging skin can be effectively improved by increasing collagen in the skin. Vitamin A (retinoic acid substance) and other active substances that can stimulate collagen production have become the main force for anti-aging. However, when vitamin A (retinoic acid substance) works in the skin, it may activate the TRPV1 pathway, causing pain and burning, and even redness, swelling and peeling. Therefore, it is imperative to prepare cosmetics that are milder and can fundamentally improve the condition of aging skin.

### SUMMARY

In view of this, the object of the present invention is to provide an anti-aging composition, skin care product and cosmetic. The anti-aging composition provided by the present invention can fundamentally improve the condition of aging skin, and is mild and non-irritating.

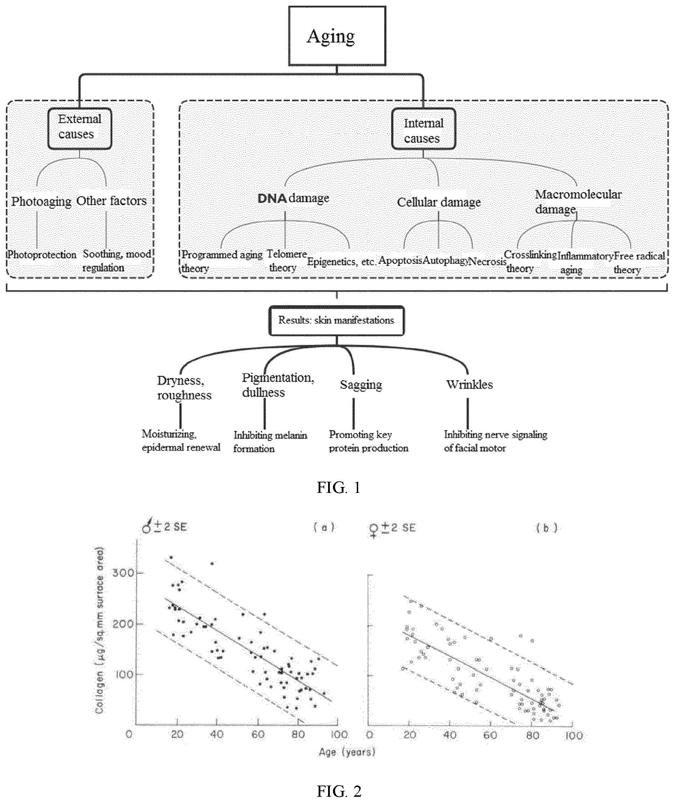

FIG. 1 is a schematic diagram of the causes and manifestations of skin aging. The causes of aging include external factors and internal factors. Among them, external factors include photoaging and other factors, such as emotions, and internal factors include DNA damage, cell damage, macromolecular damage, etc., where DNA damage has further given rise to the programmed aging theory, telomere theory, epigenetics, etc.; cell damage includes apoptosis, cell autophagy, cell necrosis, etc.; and macromolecular damage is strongly related to the cross-linking theory, inflammatory aging, free radical theory, etc. The manifestations of aging on the skin mainly include dryness, roughness, pigmentation, dullness, sagging, and wrinkles. There are different anti-aging means for different skin aging manifestations, for example, to solve the problem of dryness and roughness by moisturizing and epidermis renewal; to solve the problem of pigmentation and dullness by inhibiting the production of melanin; to solve the problem of sagging by promoting the production of key proteins; and to solve the problem of wrinkles by inhibiting the nerve signaling of facial motor.

In particular, it should be noted that the content of collagen in the skin decreases at a rate of 1% per year during the aging process (British Journal of Dermatology (1975) 93, 639), as shown in FIG. 2, which shows the change curve of collagen content in the skin with age, where FIG. 2(a) is the change curve for men, and FIG. 2(b) is the change curve for women. There are two reasons for the decline of collagen content in the skin: ① decrease in synthesis caused by decreased cell function; ② continuous degradation of collagen caused by ultraviolet rays, time and pollution. Therefore, in order to maintain a positive increase in collagen, it is necessary not only to promote its production, but also to prevent its degradation. Chronic inflammation induced by ultraviolet rays can promote the secretion of collagen-degrading enzymes and destroy key proteins in the dermis and basement membrane. Therefore, although many active substances can promote collagen synthesis, the newly synthesized collagen will be destroyed by a series of reactions caused by ultraviolet rays and inflammation, eventually making it difficult to increase the collagen content. Therefore, just promoting collagen production is not the fundamental way to solve the problem. Maintaining the continuous increase of collagen content while improving the condition of aging skin is the key means to delay skin aging.

In view of the above problems, the present invention provides an anti-aging composition, comprising *Chlamydomonas* extract and acetyl hexapeptide-8 in a mass ratio of 1:2000-5:2.

At present, synthetic peptides are commonly used raw materials for cosmetics, and the spatial structures of synthetic peptides determine their functions. Therefore, the function of synthetic peptides is usually extremely specific. Acetyl hexapeptide-8 is a well-known signal peptide (Argireline), and designed in imitation of botulinum toxin. Since it can competitively replace SNAP-25 (synapse-associated protein) to inhibit the transmission of motor nerve signals, and reduce wrinkles caused by muscle contraction, it is widely used in anti-wrinkle products. However, this ingredient can only inhibit the dynamic wrinkles caused by muscle movement, and its effect is affected by the concentration. Besides, acetyl hexapeptide-8 molecule will be metabolized by the human body soon after it acts, which makes it difficult to produce other beneficial effects on the skin, such as promoting key protein production, improving firmness, and brightening skin tone.

The inventors of the present application creatively found that the combined use of acetyl hexapeptide-8 and *Chlamydomonas* extract can inhibit inflammation, promote cell autophagy, and inhibit collagen degradation, and can continuously promote collagen production and reduce the secretion of destructive proteases and inflammatory factors, finally realizing the increase of collagen, thereby fundamentally improving the condition of aging skin.

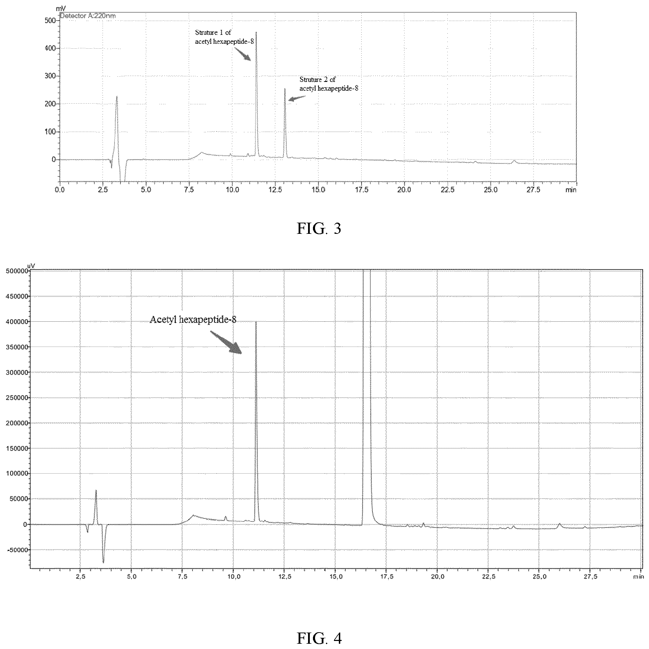

In one embodiment, the acetyl hexapeptide-8 is acetyl hexapeptide-8 with a trade name of Rheolique. Compared with other acetyl hexapeptide-8, Rheolique contains two structures of acetyl hexapeptide-8, while common commercially available acetyl hexapeptide-8 only contains one structure of polypeptide, referring to FIG. 3 and FIG. 4, where FIG. 3 is the high performance liquid chromatogram of Rheolique and FIG. 4 is the high performance liquid chromatogram of JYMed^{™} AGE botulinum hexapeptide. The combined use of acetyl hexapeptide-8 with a trade name of Rheolique and *Chlamydomonas* extract can enhance the anti-aging effect.

In one embodiment, the *Chlamydomonas* extract is an extract of *Chlamydocapsa* sp CCryo 101-99, which is extracted by: culturing *Chlamydocapsa* sp CCryo 101-99 bacterial strain in two stages, harvesting the snow algae at the second stage when the snow algae turns red, subjecting the harvested algae to high-pressure homogenization, and then mixing it with liposomes to obtain *Chlamydomonas* extract. Reference can be made to the record in US8206721B2.

In one embodiment, the *Chlamydomonas* extract is a *Chlamydomonas* extract with a trade name of Acti-Klotho. Acti-Klotho is a *Chlamydomonas* extract obtained by: collecting primordial algae samples from Spitzbergen, Norway, then cultivating the algae as raw materials, collecting red algae after cultivation, subjecting the collected algae to high-pressure homogenization and wall-breaking, and mixing it with liposomes to form *Chlamydomonas* extract. Acti-Klotho can up-regulate the expression of klotho gene and activate AMPK, thereby enhancing barrier, lightening aging spots and smoothing skin. Moreover, the combined use of Rheolique and Acti-Klotho can improve problems in facial skin such as dryness, pigmentation, roughness, sagging, dullness and wrinkles.

In one embodiment, the *Chlamydomonas* extract and the acetyl hexapeptide-8 are in a mass ratio of 1:2000-5:2. In one embodiment, the *Chlamydomonas* extract and the acetyl hexapeptide-8 are in a mass ratio of 1:1200-5:2. In one embodiment, the *Chlamydomonas* extract and the acetyl hexapeptide-8 are in a mass ratio of 1:500-5:2. In one embodiment, the *Chlamydomonas* extract and the acetyl hexapeptide-8 are in a mass ratio of 1:350-1:1. In one embodiment, the *Chlamydomonas* extract and the acetyl hexapeptide-8 are in a mass ratio of 1:7-2:7.

This application creatively uses *Chlamydomonas* extract and acetyl hexapeptide-8 in combination, especially Rheolique and Acti-Klotho. For one thing, it overcomes the shortcomings of using acetyl hexapeptide-8 alone, and can not only inhibit the dynamic wrinkles caused by muscle movement, but also promote cells to produce key collagen, filling the dermis, strengthening the basement membrane, finally improving the firmness of the skin. For another thing, it overcomes the functional limitations of *Chlamydomonas* extract, and has an inhibitory effect on skin sagging, dullness and increase in wrinkles during the aging process, thereby improving skin sagging and dullness and reducing facial wrinkles. More importantly, the combined use of *Chlamydomonas* extract and acetyl hexapeptide-8 in this application can not only promote the synthesis of collagen, but also inhibit the expression of inflammatory genes, promote the expression of autophagy genes, and resist collagen degradation and damage caused by ultraviolet rays and the like to always maintain a positive increase in collagen to delay the problems caused by the loss of collagen over time, thereby achieving long-term anti-aging effect. In addition, the composition provided by the present invention has good skin feeling, does not cause irritation to the skin, and is suitable for long-term care of various types of skin.

The anti-aging composition provided by the present invention can be used to prepare skin care products or cosmetics. Specifically, the present invention also provides a skin care product comprising the anti-aging composition described in the above technical solution.

In one embodiment, the skin care product comprises 2% to 30% of the anti-aging composition. In one embodiment, the skin care product comprises 5% to 25% of the anti-aging composition. In one embodiment, the skin care product comprises 10% to 20% of the anti-aging composition.

Specifically, the skin care product may be toner, essence, facial cream, lotion, etc. In addition to the above-mentioned anti-aging composition, this application has no special restrictions on other ingredients, which may include active ingredients with efficacy of moisturizing, whitening, desensitizing, and soothing, and may also include excipients such as a skin feeling adjusting agent, a preservative, and a thickening agent. Those skilled in the art can make selections according to the dosage form of skin care products.

In one embodiment, the skin care product comprises:
0.01wt%-5wt% of *Chlamydomonas* extract;
2wt%-20wt% of acetyl hexapeptide-8;
0.5wt%-5wt% of glycerin;
1wt%-10wt% of 1,3-propanediol;
0.001wt%-1wt% of xanthan gum;
1wt%-10wt% of caprylic/capric triglyceride;
0.1wt%-5wt% of hydrogenated lecithin;
0.01wt%-5wt% of cetostearyl alcohol;
0.01wt%-5wt% of SEPIPLUS^{™} 400;
0.1wt%-5wt% of p-hydroxyacetophenone;
0.1wt%-5wt% of 1,2-hexanediol;
and deionized water for balance.
In one embodiment, the skin care product comprises:
0.1wt%-4wt% of *Chlamydomonas* extract;
2.5wt%-10wt% of acetyl hexapeptide-8;
1wt%-4wt% of glycerin;
2wt%-8wt% of 1,3-propanediol;
0.01wt%-0.8wt% of xanthan gum;
2wt%-8wt% of caprylic/capric triglyceride;
0.5wt%-4wt% of hydrogenated lecithin;
0.1wt%-3wt% of cetostearyl alcohol;
0.1wt%-3wt% of SEPIPLUS^{™} 400;
0.5wt%-4wt% of p-hydroxyacetophenone;
0.5wt%-4wt% of 1,2-hexanediol;
and deionized water for balance.

The present invention also provides a cosmetic, comprising the anti-aging composition described in the above technical solution.

In one embodiment, the cosmetic comprises 2% to 30% of the anti-aging composition. In one embodiment, the cosmetic comprises 5%-25% of the anti-aging composition. In one embodiment, the cosmetic comprises 10%-20% of the anti-aging composition.

Specifically, the cosmetic may be toner, essence, skin lotion, facial cream, facial mask, loose powder, pressed powder, liquid foundation, cream foundation, BB cream, isolation cream, repairing cream, paste concealer, liquid concealer, contouring powder, highlighting powder or CC cream. In addition to the above-mentioned anti-aging composition, this application has no special restrictions on other ingredients, which may include active ingredients with efficacy of moisturizing, whitening, desensitizing, and soothing, and may also include excipients such as a skin feeling adjusting agent, a preservative, and a thickening agent. Those skilled in the art can make selections according to the dosage form of skin care products.

This application creatively uses *Chlamydomonas* extract and acetyl hexapeptide-8 in combination, especially Rheolique and Acti-Klotho, and has advantages of: ① inhibiting chronic inflammatory response in the skin (caused by age and ultraviolet ray damage) by inhibiting the expression of inflammatory genes, reducing pigmentation caused by inflammation, thereby improving skin gloss; ② promoting the expression of cell autophagy genes, removing intracellular damage and aging organelles, improving cell function, thereby maintaining collagen production; ③ down-regulating the expression of destructive proteases (MMPs) genes, reducing the degradation of collagen, and finally realizing the increase of collagen. In addition, when the compositions provided by the present invention are applied to formulations, they have good skin feeling, do not cause irritation to the skin, and are suitable for long-term care of various types of skin.

### BRIEF DESCRIPTION OF DRAWINGS

FIG. 1 is a schematic diagram of the causes and manifestations of skin aging;
FIG. 2 shows the change curve of collagen content in the skin with age;
FIG. 3 is a high performance liquid chromatogram of Rheolique in the composition provided by the example of the present invention;
FIG. 4 is a high performance liquid chromatogram of a common acetyl hexapeptide-8 in the composition provided by the example of the present invention.

### DETAILED DESCRIPTION

The anti-aging composition, skin care product and cosmetic provided by the present invention will be described in detail below in conjunction with the examples.

In the following examples, the acetyl hexapeptide-8 with a trade name of Rheolique has a high performance liquid chromatogram as shown in FIG. 3. FIG. 3 shows the high performance liquid chromatogram of Rheolique in the composition provided by the examples of the present invention. The commercially available common acetyl hexapeptide-8 with a trade name of JYMed^{™} AGE botulinum hexapeptide was purchased from JYMed Technology Co., Ltd., and has a high-performance liquid chromatogram as shown in FIG. 4. FIG. 4 shows the high performance liquid chromatogram of the common acetyl hexapeptide-8 in the composition provided by the examples of the present invention. The *Chlamydomonas* extract with a trade name of Acti-Klotho was purchased from mibelle group, and was obtained by collecting primordial algae from Spitzbergen, Norway, then cultivating the algae. The commercially available common algae extract with a trade name of Seamollient was purchased from Jinxing Trading Co.,Ltd..

### Examples 1-9

Anti-aging compositions were prepared according to the formula shown in Table 1 (by mass percentage):

**Table 1 Formula of the anti-aging compositions provided by Examples 1-9 of the present invention**

| Example | 1 | 2 | 3 | 4 | 5 | 6 | 7 | 8 | 9 |
|---|---|---|---|---|---|---|---|---|---|
| Acti-Klotho | 0.5% | 1% | 5% | 2% | 1% | 0.01% | 0.01% | 0.01% | 3% |
| Rheolique | 3.5% | 10% | 2% | 2% | 3.5% | 5% | 12% | 18% | 1% |
| Normal saline | 96% | 88% | 93% | 96% | 95.5% | 94.99% | 87.99% | 81.99% | 96% |

Anti-aging compositions were prepared according to the formula shown in Table 2 (by mass percentage):

**Table 2 Formula of the anti-aging compositions provided by the Comparative examples 1-9 of the present invention**

| Comparative example | 1 | 2 | 3 | 4 | 5 | 6 | 7 | 8 | 9 | 10 |
|---|---|---|---|---|---|---|---|---|---|---|
| Acti-Klotho | 0.5% | / | 1% | / | 0.5% | / | / | 1% | / | 1% |
| Rheolique | / | 3.5% | / | 4% | | 3.5% | / | / | 3.5% | / |

| | | | | | | | | | | |
|---|---|---|---|---|---|---|---|---|---|---|
| Common commercially available acetyl hexapeptide-8 | / | / | / | / | 3.5% | | 3.5% | 3.5% | / | 3.5% |
| Common commercially available algae extract | / | / | / | / | / | 0.5% | | / | 1% | 1% |
| Normal saline | 99. 5% | 96.5% | 99% | 96% | 96% | 96% | 96% | 95.5% | 95.5% | 95.5% |

The preparation method is as follows: algae extract and acetyl hexapeptide-8 were dissolved in normal saline at 25°C with stirring for 30 min, and mixing the mixture evenly to obtain an anti-aging composition.

### Test Example 1 Genome test:

The anti-aging compositions prepared in Examples 1-9 and Comparative Examples 1-10 were applied to the explant skin test model for 48 h of culture, then the test substance was removed, and samples were taken from the explant skin for analysis of the gene transcription level.

Model culture: Source of isolated skin tissue for testing: skin explants (2 cm×2 cm) from abdominal plastic surgery (49-year-old female). Medium: medium for maintaining human isolated skin. Culture conditions: 37°C, 5% carbon dioxide, humid air.

Model processing and preparation: The compositions of the above examples and comparative examples were systemically (20 mg/cm²) applied on isolated skin for 48 h of culture, during which the compositions were renewed after 24 h. The isolated skin without any treatment was used as a control. Three parallels were set up for each sample. After the culture was completed, the isolated skin was washed with phosphate-buffered saline (PBS) solution. Then each explant was perforated (8 mm in diameter) 3 times, and immediately stored in RNA preservation solution at 4°C for 24 h and then frozen at -80°C for later use.

Gene expression analysis test: Samples were subjected to automatic homogenization with Precellys/cryolys, the total RNA of each sample was extracted using the NucleoSpin RNA kit, and the total RNA quality of the sample was detected by capillary electrophoresis and spectrophotometer. Labeled and amplified anti-sens RNA (aRNA) was obtained from each RNA sample using the GeneChip 3'TVT PLUS kit (Affymetrix). Each labeled and amplified aRNA sample was evaluated with the pre- and post- spectra of the fragments by using capillary electrophoresis. The aRNA fragments were hybridized with the Affymetrix U219 chip at the GeneAtlasTM fluidics Affymetrix hybridization station for 20 h (45°C). Data were normalized with the RMA algorithm using software Expression Console (Affymetrix). The control group was taken as the baseline, and the test values of the control group were subtracted from the test results of all experimental groups to obtain the final result. "+" represents an increase compared with the control group, and "-" represents a decrease compared with the control group.

The experimental results are shown in Table 3 and Table 4. Table 3 shows the gene transcription level in the explanted skin model provided by the examples of the present invention, and Table 4 shows the gene transcription level in the explanted skin model provided by the comparative examples of the present invention.

**Table 3 Gene transcription level in the explanted skin model provided by the examples of the present invention**

| Function | Transcript ome gene name | Exam ple 1 | Exam ple 2 | Examp le 3 | Exam ple 4 | Exam ple 5 | Exam ple 6 | Exam ple 7 | Exam ple 8 | Exam ple 9 |
|---|---|---|---|---|---|---|---|---|---|---|
| Inflammat ion related gene | IL-33 | -45%* | -41% * | -32%* | -37%* | -54%* | -34%* | -30%* | -28%* | -21%* |
| | ST2 | -22%* | -21% * | -16%* | -19%* | -31%* | -17%* | -18%* | -15%* | -12%* |
| Autophag y related gene | ULK1 | +35% * | +30% * | +22%* | +25% * | +49% * | +23% * | +20% * | +19% * | +16% * |
| | ATG13 | +32% * | +24% * | +17%* | +21% * | +47% * | +19% * | +16% * | +16% * | +14% * |
| | AMBRA1 | +33% * | +28% * | +18%* | +20% * | +45% * | +18% * | +15% * | +16% * | +13% * |
| | ATG4D | +26% * | +24% * | +20* | +23% * | +43% * | +21% * | +19* % | +17* % | +15* % |
| | LAMP3 | +53% * | +45% * | +31%* | +42% * | +67% * | +36% * | +29% * | +26% * | +22% * |
| Collagen destroying enzyme gene | MMP-1 | -58%* | -0.46 %* | -36%* | -0.41 %* | -66%* | -0.38 %* | -35%* | -33%* | -30%* |
| | MMP-3 | -53%* | -49% * | -36%* | -44%* | -62%* | -40%* | -38%* | -35%* | -29%* |
| | MMP-9 | -75%* | -66% * | -51%* | -61%* | -86%* | -54%* | -48%* | -44%* | -32%* |

**Table 4 Gene transcription level in the explanted skin model provided by the comparative examples of the present invention**

| Functi on | Transcr iptome gene name | Comp arativ e examp le 1 | Comp arativ e examp le 2 | Comp arativ e examp le 3 | Comp arativ e examp le 4 | Comp arativ e examp le 5 | Comp arativ e examp le 6 | Comp arativ e examp le 7 | Comp arativ e examp le 8 | Comp arativ e examp le 9 | Comp arativ e examp le 10 |
|---|---|---|---|---|---|---|---|---|---|---|---|
| Inflam mation related gene | IL-33 | -6% | -4% | -10% | -8% | -6% | -5% | -6% | -4% | -9% | -4% |
| | ST2 | -1% | -4% | -5% | -7% | -3% | -6% | -4% | -5% | -8% | -4% |
| Autop hagy related gene | ULK1 | +4% | +2% | +5% | +5% | +6% | +3% | -3% | -2% | +5% | -3% |
| | ATG13 | +2% | +3% | +5% | +2% | +3% | +5% | +4% | +5% | +3% | +4% |
| | AMBR A1 | -2% | +3% | +4% | +5% | +1% | +6% | +4% | +5% | +5% | +4% |
| | ATG4 D | +4% | +2% | +7% | +5% | +5% | +2% | +3% | +3% | +5% | +1% |
| | LAMP 3 | +2% | +2% | +8% | +1% | +5% | +3% | +1% | -1% | +3% | -2% |
| Collag en destro ying enzym e gene | MMP-1 | -4% | -7% | -8% | -9% | -6% | -7% | -1% | -4% | -9% | -2% |
| | MMP-3 | -6% | -3% | -7% | -8% | -2% | -6% | -4% | -6% | -7% | -5% |
| | MMP-9 | -5% | -2% | -9% | -6% | -4% | -4% | -7% | -5% | -4% | -3% |

| | | | | | | | | | | | |
|---|---|---|---|---|---|---|---|---|---|---|---|
| In Table 3, * represents significant analysis p<0.05. | | | | | | | | | | | |

As shown in Table 3, the compositions provided by the present application can down-regulate inflammation genes and matrix metalloproteinase genes, and up-regulate autophagy genes. Inflammation genes participate in and promote the occurrence of chronic inflammation in the skin, matrix metalloproteinases can directly degrade collagen, and autophagy genes promote the process of cell autophagy, which can remove intracellular damage and aging organelles, and provide cells with transformed nutrients. Therefore, the composition provided by the present invention can inhibit inflammatory response, promote cell autophagy, and inhibit collagen degradation. In conjunction with the following detection at the cellular level, these processes are of great significance for maintaining the increase of collagen.

### Test Example 2 Test and analysis of cell model

### Cell culture:

Human fibroblasts (primary) in good condition required for the test were prepared, 200 µL of cell suspension was inoculated in each well in a 6-well plate, and 2 mL of complete medium was added to each well. The plate was put in a carbon dioxide incubator at 37°C, 90% RH and 5% CO₂;

### Administration:

The compositions of the examples and comparative examples required for the test were prepared, and added to the sample wells at 200 µL/well. The cells were incubated for 24-48 h. 200 µL of the cell supernatant was added into a 1.5 mL centrifuge tube, and centrifuged at 5000 RPM for 10 min, and the supernatant was collected for later use. The content of extracellular secretion was detected by a kit.

### Elisa test

Collagen type I, inflammatory factor IL-1α, and destructive protease MMP-1 were all detected by kits, respectively according to "Human Type I Collagen α1 ELISA Kit Instruction Manual", "Human Matrix Metalloproteinase-1 ELISA Kit Instruction Manual" and "Human Interleukin 1α ELISA Kit Instructions".

The test results are shown in Table 5, which shows the protein expression levels in the cell models provided in the examples and comparative examples of the present invention.

**Table 5 Protein expression levels in the cell models provided in the examples and comparative examples of the present invention**

| Test indicator s | Cult ure time | Exa mple 1 | Exa mple 2 | Exa mple 3 | Exa mple 4 | Exa mple 5 | Exa mple 6 | Compar ative exampl e5 | Compar ative example 6 | Compar ative exampl e7 | Compar ative exampl e8 |
|---|---|---|---|---|---|---|---|---|---|---|---|
| Collage n type I change rate | Cult ure for 12 h | +41 %^{∗} | +35 %^{∗} | +25 %^{∗} | +29 %^{∗} | 52% ^{∗} | +28 %^{∗} | +13% | +19% | 15% | +21% |
| | Cult ure for 24 h | +59 %^{∗} | +48 %^{∗} | +37 %^{∗} | +45 %^{∗} | 65% ^{∗} | +42 %^{∗} | +11% | 15% | 11% | 17% |
| | Cult ure for 30 h | +82 %^{∗} | +56 %^{∗} | +42 %^{∗} | +51 %^{∗} | 71% ^{∗} | +48 %^{∗} | -7% | -8% | -6% | -4% |
| Destruct ive protease | MM P-1 | -58% ^{∗} | -32% ^{∗} | -26% ^{∗} | -30% ^{∗} | -65% ^{∗} | -32% ^{∗} | -15% | -13% | -11% | -18% |
| Inflamm atory factors | IL-1 α | -64% ^{∗} | -52% ^{∗} | -39% ^{∗} | -47% ^{∗} | -75% ^{∗} | -46% ^{∗} | -6% | -7% | -4% | -9% |

| | | | | | | | | | | | |
|---|---|---|---|---|---|---|---|---|---|---|---|
| Among them, * represents significant analysis p<0.05. | | | | | | | | | | | |

As shown in Table 5, the compositions provided by the present application can continuously promote the production of collagen within 30 h, and reduce the secretion of destructive proteases and inflammatory factors. Although the compositions provided in Comparative Examples 5-8 can promote collagen production in a short period of time, it is difficult for them to sustain the production. In conjunction with the results of Test Example 1, this may be due to the fact that the destructive proteases (matrix metalloproteinases MMPs) in the comparative examples increased over time, which continuously degraded collagen, eventually leading to a decrease in collagen. In contrast, the compositions in the examples can promote collagen production, reduce collagen degradation, and ultimately realize increase in collagen through three dimensions: inhibiting the expression of inflammatory genes, down-regulating destructive protease levels, and promoting cellular autophagy.

### Examples 10-11

Anti-aging compositions were prepared according to the formula shown in Table 6 (by mass percentage):

**Table 6 Formula of the anti-aging compositions provided by Examples 10-11 and Comparative Example 11 of the present invention**

| Phase | Raw material name | Example 10 | Example 11 | Comparative example 11 |
|---|---|---|---|---|
| A | *Chlamydomonas* extract (Acti-klotho) | 0.5 | 1 | / |
| A | Acetyl hexapeptide-8 (Rheolique) | 3.5 | 3.5 | / |
| A | Common commercially available acetyl hexapeptide-8 | / | / | 0.5 |
| A | Common commercially available algae extract | / | / | 3.5 |
| A | Glycerin | 2 | 2 | 2 |
| B | Deionized water | 80.68 | 80.18 | 80.68 |
| B | 1,3-Propanediol | 5 | 5 | 5 |
| B | Xanthan gum | 0.02 | 0.02 | 0.02 |
| C | Caprylic/capric triglyceride | 5 | 5 | 5 |
| C | Hydrogenated lecithin | 0.5 | 0.5 | 0.5 |
| C | Cetostearyl alcohol | 1.0 | 1.0 | 1.0 |
| C | SEPIPLUS^{™} 400 | 0.8 | 0.8 | 0.8 |
| D | p-Hydroxyacetophenone | 0.5 | 0.5 | 0.5 |
| D | 1,2-Hexanediol | 0.5 | 0.5 | 0.5 |

The preparation method is as follows:
(1) All the raw materials of phase B were weighed, stirred, heated to 85°C, and mixed well to obtain an aqueous phase; and all the raw materials of phase C were weighed, stirred, heated to 80°C, and mixed well to obtain an oil phase;
(2) The oil phase raw material was poured into the water phase, and homogenized at 7000 rpm×5 min to obtain a crude emulsion;
(3) The raw materials of phase D were heated (85°C) and melted to form a uniform transparent liquid, which was then added to the crude emulsion described in (2), and the mixture was stirred and cooled to 45°C;
(4) The raw materials of phase A were weighed, stirred at room temperature, mixed evenly, and added to the emulsion described in (3), and the mixture was stirred evenly to obtain an emulsion product comprising the anti-aging composition.

### Test Example 3 Clinical efficacy test

Commercially available SkinCeuticals Hyaluronic acid intensifier was used for Comparative Example 12.

Referring to the human efficacy test method of SGS Standard Technical Services Co., Ltd., a total of 42 healthy volunteers were recruited, the number of people included was 40, the number of people who failed to complete the test was 2, and the number of people with valid data analysis was 40.

Inclusion criteria: (1) Healthy female subjects aging 19-40 years old; (2) with dull facial complexion lacking luster, sagging skin lacking firmness, visible fine wrinkles around the eyes; (3) being able to cooperate well with the experimenters and maintain the regularity of life during the research period; (4) without serious systemic diseases, immune deficiency or autoimmune diseases; (5) being able to read and understand all the contents of the informed consent form, and voluntarily sign the informed consent form (6) only using designated skin care samples during the test; (7) no participation in the clinical trials of any other research centers during the test period; (8) agreeing not to apply any cosmetics, drugs and health products that affect the results during the test period; (9) avoiding sun exposure during the test period; (10) avoiding excessive exercise during the test period; (11) no drinking alcohol before each return visit.

Exclusion criteria: Anyone who has any of the following conditions must be excluded from this study: (1) those with skin diseases on the face that may affect the judgment of the test results; (2) those with high allergies; (3) those with a history of cosmetic allergies; (4) women who are pregnant, breast-feeding or planning to become pregnant during the test; (5) those with severe heart, liver, kidney function damage and severe immunocompromise; (6) those with mental illness and severe endocrine disease, and oral contraceptive users (7) those with significant nutritional disorders; (8) those with drug or alcohol intoxication; (9) those who have used similar cosmetics and pharmaceuticals on the same test site for no more than 3 months; (10) those who have accepted skin treatments, cosmetics, and other tests that may affect the results.

Withdrawal criteria: (1) Subjects who voluntarily request to withdraw; (2) subjects with poor compliance, unable to use samples and cooperate with the test as required; (3) subjects who use other samples with the same efficacy as the research samples during the test; (4) subjects who have adverse reactions during the test and are unable to continue the test.

The subjects were randomly divided into two groups, Example 10 (A) and Comparative Example 11 (B) were one group, and Example 11 (B) and Comparative Example 12 (A) were another group. The subjects needed to apply the products A and B to be tested on the upper half of the left and right sides of the face respectively, and use them every morning and evening for 28 consecutive days, and the skin condition of the volunteers before and after use was tested. In terms of the index change rate before and after using the product, the test results are shown in Table 7, which shows the efficacy test results of the cosmetics provided in the examples and comparative examples of the present application.

**Table 7 Efficacy test results of the cosmetics provided by the examples and comparative examples of the present application**

| Function | Specific indicators | Example 10 | Example 11 | Comparative example 11 | Comparative example 12 |
|---|---|---|---|---|---|
| Moisturizing | Moisture content in stratum corneum | +12.8%^{∗} | +13.8%^{∗} | +7.9%^{∗} | +12.14%^{∗} |
| Brightening | Skin gloss value | +9.78%^{∗} | +10.26%^{∗} | +5.08% | +8.78% |
| Firming | Skin elasticity parameters (the lower the F4, the firmer the skin) | -0.78%^{∗} | -0.88%^{∗} | -0.60% | +0.63% |
| Anti-wrinkle | Volume of wrinkles under the eyes | -27.01%^{∗} | -29.05%^{∗} | -2.82% | +16.61% |
| | Area of wrinkles under the eyes | -12.29%^{∗} | -25.51%^{∗} | -3.68% | -0.25% |
| | Average depth of wrinkles under the eyes | -42.15%^{∗} | -52.34%^{∗} | -14.83% | +9.97% |
| Skin feeling favorability | Subject rating (out of 10 points) | 8.2 | 8.6 | 7.4 | 6.7 |
| Side effect | Redness, swelling, peeling and the like | None | None | None | Slight tingling |

| | | | | | |
|---|---|---|---|---|---|
| In Table 7, * represents significant analysis p<0.05, indicating a significant change from before use. | | | | | |

As shown in Table 7, when the compositions provided by the present invention were applied in formulations, they can effectively brighten the skin tone, improve skin firmness, and remove wrinkles, and the effect was obviously better than those of Comparative Examples 11 and 12.

In conjunction with the genetic analysis and protein test results, it is found that skin gloss was affected by inflammation in the skin, and skin elasticity parameters and wrinkles were negatively correlated with collagen content. The compositions and cosmetics provided by the present invention are capable of ① inhibiting chronic inflammatory response in the skin (caused by age and ultraviolet ray damage) by inhibiting the expression of inflammatory genes, reducing pigmentation caused by inflammation, thereby improving skin gloss; ② promoting the expression of cell autophagy genes, removing intracellular damage and aging organelles, improving cell function, thereby maintaining collagen production; ③ down-regulating the expression of destructive proteases (MMPs) genes, reducing the degradation of collagen, and finally realizing the increase of collagen. In addition, when the compositions provided by the present invention are applied to formulations, they have good skin feeling, do not cause irritation to the skin, and are suitable for long-term care of various types of skin.

The above are only preferred embodiments of the present invention, but the scope of protection of the present invention is not limited thereto. Within the technical scope disclosed in the present invention, those skilled in the art can make equivalent replacement or change according to the technical solution and the inventive concept of the present invention, which shall fall within the protection scope of the present invention.

## Claims

1. An anti-aging composition, comprising *Chlamydomonas* extract and acetyl hexapeptide-8 in a mass ratio of 1:2000-5:2.

2. The anti-aging composition according to claim 1, wherein the *Chlamydomonas* extract is an extract of *Chlamydocapsa* sp CCryo 101-99.

3. The anti-aging composition according to claim 1, wherein the *Chlamydomonas* extract is a *Chlamydomonas* extract with a trade name of Acti-Klotho.

4. The anti-aging composition according to any one of claims 1-3, wherein the acetyl hexapeptide-8 is acetyl hexapeptide-8 with a trade name of Rheolique.

5. The anti-aging composition according to claim 4, wherein the *Chlamydomonas* extract and the acetyl hexapeptide-8 are in a mass ratio of 1:1200-5:2, preferably 1:500-5:2, more preferably 1:350-1:1, most preferably 1:7-2:7.

6. Use of the anti-aging composition according to any one of claims 1 to 5 in the production of a skin care product or cosmetic.

7. A skin care product, comprising the anti-aging composition according to any one of claims 1-5.

8. The skin care product according to claim 7, comprising 2%-30% of the anti-aging composition.

9. The skin care product according to claim 8, comprising:
0.01 wt%-5wt% of *Chlamydomonas* extract;
2wt%-20wt% of acetyl hexapeptide-8;
0.5wt%-5wt% of glycerin;
1wt%-10wt% of 1,3-propanediol;
0.001wt%-1wt% of xanthan gum;
1wt%-10wt% of caprylic/capric triglyceride;
0.1wt%-5wt% of hydrogenated lecithin;
0.01wt%-5wt% of cetostearyl alcohol;
0.01wt%-5wt% of SEPIPLUS^{™} 400;
0.1wt%-5wt% of p-hydroxyacetophenone;
0.1wt%-5wt% of 1,2-hexanediol;
and deionized water for balance.

10. A cosmetic comprising the anti-aging composition according to any one of claims 1-5.
